# EUROPEAN PATENT SPECIFICATION

(11) **EP 1 366 715 B1**
(45) Date of publication and mention of the grant of the patent: **02.02.2005**
(21) Application number: 03011896.2
(22) Date of filing: 27.05.2003
(51) Int. Cl.: A61B 10/00, B01L 3/14, B01L 3/00

(54) **Extraction tube for collection of faeces samples**
Auszugsröhre zum Sammeln von Stuhlproben
Tube d'extraction pour la collection d'échantillons de féces

(30) Priority: 31.05.2002 IT MI20020287 U
(43) Date of publication of application: 03.12.2003
(73) Proprietor: Sentinel CH. S.r.L., 20155 Milano (IT)
(72) Inventor: De Luca, Ugo, 20144 Milano (IT); Roveda, Luigi, 22030 Caglio (Como) (IT); Fornari, Filpppo, 20145 Milano (IT)
(74) Representative: Gervasi, Gemma, Dr.

(56) References cited:
- EP-A- 0 727 653
- US-B1- 6 299 842

## Description

The present invention refers to an extraction and sampling device for collection of faeces samples, particularly for laboratory immunological tests, for qualitative or quantitative determination of one or more analytes, such as haemoglobin, Helicobacter pylori and the like.

At present, the following two methods, among others, are known for testing for analytes present in faeces samples: the chemical method wherein a defined amount of the sample collected is added to a reagent constituting an active principle which reacts chemically with the analyte under examination giving rise to a chromogenic reaction indicative of the presence of the analyte and the immunological or immunochromographic method wherein the sample collected is dissolved in a buffer solution which is dropped onto a diagnostic device containing an absorbent membrane treated with specific antibodies bound to coloured substances which fix the analytes under examination giving rise to a coloured sign on the membrane.

These tests are done manually by the operator and are of a qualitative type; that is to say, they reveal the presence or absence of an analyte.

To carry out sampling of the faeces an extraction tube comprising a cylindrical container with a screw closure cap is used. In the lower inside surface of the cap there is mounted a screw small rod which protrudes axially downwards therefrom. The screw small rod has a threaded portion in its free end, destined for collection of faeces sample. In fact, when the small rod is inserted into the faeces, faeces samples are retained in its thread.

When the cap is screwed onto the mouth of the container, the screw small rod is partially immersed inside the container, which is suitably filled with a buffer solution having a suitable pH. In this manner the collected faeces sample detaches from the thread of the screw small rod and is dispersed in the buffer solution.

In the upper outside surface of the cap a breakable tip protruding axially and upward therefrom is provided. Breakage of the tip forms an outlet aperture in the cap communicating with the inside of the container. In this manner the cap acts as a dropper allowing the buffer solution - wherein faeces sample is dissolved - to drop through the outlet aperture.

During the sampling operation the patient must unscrew the cap from the container, plunge the screw small rod into the faeces so that faeces samples are retained in the thread of the screw small rod, remove with the aid of a cotton wool ball - if possible - the excess faeces accumulated on the small rod outside the thread, and lastly screw the screw cap into the container to immerse the screw small rod in the buffer solution.

Subsequently, to perform the test, the tip is broken and the buffer solution containing the sample is dropped onto a suitable diagnostic device which contains a nitrocellulose membrane treated with specific antibodies to the analyte (for example haemoglobin) under examination.

Said collection tube presents some drawbacks. In fact the operation of removing the excess faeces with cotton wool is quite awkward for the user. Furthermore, the outlet aperture of the dropper often becomes obstructed with consequent problems in releasing the solution. Also, the tip of the cap which closes the outlet aperture can break accidentally during handling and/or transport with the result of involuntary release of the solution containing biologically hazardous material.

The drawback of removal of excess faeces from the screw small rod is solved by known extraction tubes, which provide a dividing partition in the form of an annular flange disposed in the container above the level of the buffer solution. Said dividing flange has a central hole wherein the screw small rod is inserted so that the excess faeces is retained above the dividing flange and does not fall into the buffer solution.

In this case, the tip of the outlet aperture is disposed in the part of the container opposite the mouth that receives the cap of the screw small rod. Another already known solution provides a device similar to that described above wherein a second screw cap disposed to protect the tip is provided, to prevent accidental breakage thereof.

However, in extraction tubes of the prior art the problem of occlusion of the outlet aperture of the dropper nevertheless remains. Furthermore said extraction tubes have been designed for manual tests.

For use in automated systems, the buffer solution containing the dissolved faeces samples must be transferred to special sample-holder tubes which are labelled with identification codes and loaded into an automatic analyser which, in a per se known manner, performs the test.

It is apparent that said operation of transferring the buffer solution with faeces samples to the sample-holder tube of automatic analysers is quite tiresome for the operator concerned and involves an excessive waste of time.

EP 0 727 653 discloses an extraction tube according to the preamble of claim 1.

It is an object of the present invention to overcome the prior art drawbacks by providing an extraction and sampling tube for the collection of faeces samples that is practical for the patient and simple to use for the laboratory technician.

Another object of the present invention is to provide an extraction and sampling tube for the collection of faeces samples that is versatile and suitable to be applied directly to different test systems present on the market, such as automatic clinical chemistry and immunological test analysers.

Yet another object of the present invention is to provide an extraction and sampling tube for faeces collection that is economical and simple to make.

These objects are achieved according to the invention with the characteristics listed in appended independent claim 1.

Advantageous embodiments of the invention are apparent from the dependent claims. The extraction tube for collection of faeces samples, according to the invention, comprises a container body hollow on the inside for containing a buffer solution and faeces samples, a top cap applied to the top end of the container body and provided with a threaded small rod for sampling of faeces samples and a bottom cap applied removably to the bottom end of the container body.

The main characteristic of the present invention is that said extraction tube can be used directly for automatic tests.

In the extraction tube according to the invention the breakable tip provided by the prior art extraction tubes is eliminated and, consequently, all the drawbacks due to the breakable tip are eliminated.

Furthermore, the upper part of the top cap of the extraction tube according to the invention is advantageously substantially dome-shaped, similarly to the bottom of a primary sampling tube, which makes its use possible on automatic clinical chemistry and immunological test analysers. The diameter of the container body of the extraction device according to the invention is also substantially equal to the diameter of the sample tubes routinely used in laboratories. Consequently the extraction tube according to the invention can be used directly on automatic clinical chemistry and immunological test analysers, without the need of transferring the contents thereof to special test tubes suitable to be positioned in the sample-holder plate of said automatic analysers.

Further characteristics of the invention will be made clearer by the following detailed description, referring to a purely exemplary and therefore non-limiting embodiment thereof, illustrated in the appended drawings, in which:
Figure 1 is an exploded front view illustrating the extraction tube for collection of faeces samples according to the invention;
Figure 2 is an axial sectional view along the plane of section plane II-II of Figure 1;
Figure 3 is an axial sectional view illustrating the extraction tube according to the invention assembled and with a buffer solution therein;
Figure 4 is an axial sectional view illustrating the extraction tube according to the invention, ready for use in a support of an automatic machine for clinical chemistry and immunological tests, wherein the support is shown broken off.

The extraction tube for collection of faeces samples according to the invention, designated as a whole with reference numeral 1, is described with the aid of the figures.

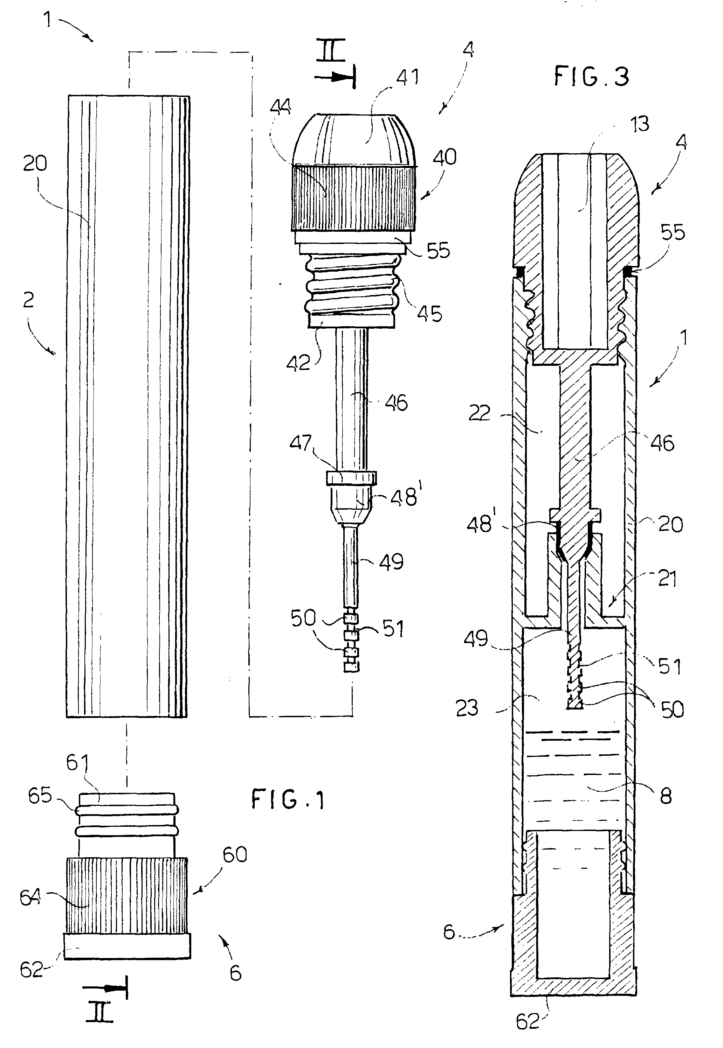

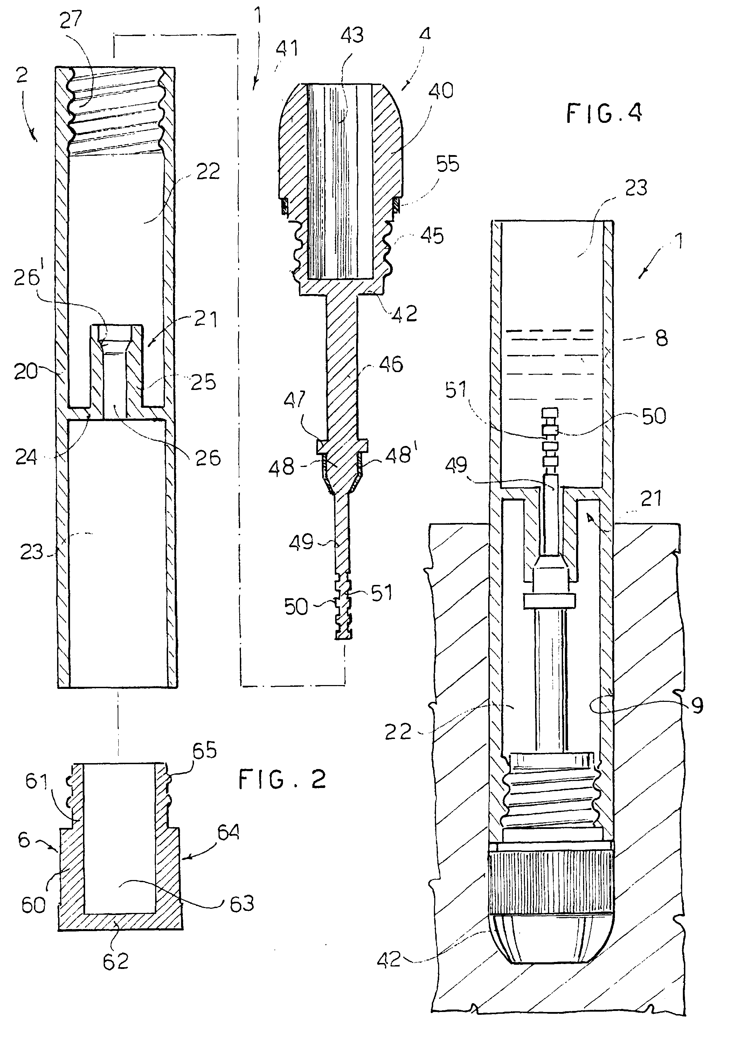

In particular, with reference to Figures 1 and 2 the extraction tube 1 which comprises a container 2, a top cap 4 and a bottom cap 6 is shown exploded.

The container 2 comprises a substantially cylindrical body 20, hollow on the inside and open at the top and at the bottom. Inside the body 20 of the container, in an intermediate position, a dividing partition 21 is provided which divides the inside of the container 2 into a top chamber 22 and a bottom chamber 23.

The dividing partition 21 comprises an annular flange 24 which protrudes radially inwards. A cylindrical tang 25 protrudes axially upward from the top of the annular flange 24. The dividing partition 21 has an axial through hole 26 which passes axially through the cylindrical tang 25 and the annular flange 24. The hole 26 has in its upper part a substantially tapered portion 26' with an increasing diameter with respect to the lower part.

In the inside surface of the container body 20, in the upper end of the top chamber 22, an internal thread 27 is provided.

The top cap 4 comprises a body consisting of a substantially cylindrical middle part 40 with an outside diameter substantially equal to the outside diameter of the container body 20, a substantially dome-shaped top part 41 disposed above the middle part and a substantially cylindrical bottom part 42, disposed beneath the middle part and having an outside diameter smaller than that of the middle part and slightly smaller than the inside diameter of the container body 20.

An upwardly open blind hole 43, hexagonal in shape in cross section, is provided axially in the body of the cap 4.

Knurling 44 consisting of a plurality of longitudinal ribs and grooves is provided in the outside surface of the middle part 40 of the body of the top cap 44.

An annular seal 55 is provided between the knurled part 44 and the threaded part 45 of the top cap.

An external thread 45 able to engage, in a screwing relationship, with the internal thread 27 of the container body is provided in the outer surface of the central bottom part 42.

A stem 46 having a smaller diameter than the bottom part 42 protrudes axially downward from the bottom part 42 of the top cap. At the bottom end of the stem 46 a collar or annular flange 47 which protrudes radially from the stem 46 is provided. The collar 47 has an outside diameter substantially equal to the outside diameter of the tang 25 of the dividing partition of the container 2.

A tang 48 with a smaller outside diameter than the collar 47 is provided beneath the collar 47. A seal 48' able engage sealably in the tapered part 26' of the hole 26 of the dividing partition 21 is disposed around the tang 48. A small rod 49 with a smaller diameter than the tang 48 and the hole 26 in the dividing partition 21 is provided axially beneath the tang 48.

A thread formed by a plurality of collars 50 disposed spaced apart from each other so as to form annular spaces 51 between said collars 50 is provided in the bottom portion of the small rod 49.

The bottom cap 6 comprises a body consisting of a middle cylindrical part 60 having an outside diameter substantially equal to the outside diameter of the container 2, a top cylindrical part 61 having a slightly smaller outside diameter with respect to the inside diameter of the container body 20 and a disk-shaped bottom part 62 having an outside diameter slightly greater than the outside diameter of the central part.

An upwardly open blind hole 63 is provided axially in the body of the bottom cap 6. Knurling 64 consisting of a plurality of longitudinal ribs and grooves is provided in the outer surface of the middle part 60 of the bottom cap 6.

Annular collars 65 which protrude radially outward so that the top part 61 of the bottom cap can engage by pressure in the bottom part 22 of the container are provided in the outer surface of the top part 61.

Assembly of the extraction tube 1 is described below with reference to Figure 3. The external thread 45 of the top cap 3 is screwed into the internal thread 27 of the container.

In this manner the small rod 49 of the top cap 4 enters the axial hole 26 of the dividing partition 21, until the seal 48' of the tang of the small rod settles tightly in the tapered part 26' of the hole 26 in the dividing partition 21 and at the same time the annular seal 55 of the top cap is disposed tightly on the upper edge of the container body 20. In this situation, the threaded part 50 of the small rod 49 is situated inside the bottom chamber 23 of the container, the seal 48' of the tang 48 of the small rod 49 is housed sealably inside the tapered part 26' of the hole 26 of the dividing partition and thus the top chamber 22 of the container is perfectly separated from the bottom chamber 23 of the container.

At this point a buffer solution 8 is introduced through the bottom opening of the container body 2 and then the bottom cap 6 is applied. The buffer solution 8 can be, for example, a solution with a pH between 7 and 9 with specific stabilizers. It should be noted that, by virtue of the presence of the dividing partition 21 closed by the seal 48' of the tang 48 of the stem of the top cap, the buffer solution 8 cannot pass from the bottom chamber 23 to the top chamber 22 of the container.

As shown in Figure 3, when the extraction tube 1 is in the upright position, that is to say when the bottom part 62 of the bottom cap 6 rests on a horizontal plane, the buffer solution 8 is partially inside the blind hole 63 of the bottom cap and partially in the bottom chamber 23 of the container, whilst the threaded part 50 of the small rod 49 of the top cap is not immersed in the buffer solution 8.

The user, when has to collect a faeces sample, unscrews the top cap 4 and plunges the small rod 49 into the faeces. Consequently, on extracting the small rod 49 from the faeces, faeces samples are retained in the annular spaces 51 in the thread 50 of the small rod 49, and also excess faeces remain around the small rod 49.

Subsequently the user screws the top cap 4 into the container 2. As a result the small rod 49 passes through the hole 26 in the dividing partition and the excess faeces are retained by the upper edge of the tang 25 of the dividing partition and thus remain in the top chamber 22. On the other hand, the faeces required for the analysis is retained in the annular spaces 51 of the thread 50 of the small rod.

By shaking the extraction tube 1, the buffer solution 8 immerses the threaded part 50 of the screw small rod and, as a result, the faeces samples are dispersed in the buffer solution. At this point the extraction tube 1 can be used as a primary sampling tub to be inserted in the sample-holder plate of automatic clinical chemistry and immunological test analysers.

As shown in Figure 4, in the extraction tube 1, in the upturned position, the buffer solution 8 occupies the bottom chamber 23 and totally immerses the threaded part 50 of the small rod 49. It should be noted that, even in this situation, due to the presence of the dividing partition 21 which is closed by the seal 48' of the tang of the small rod, the buffer solution 8 cannot pass from the bottom chamber 23 to the top chamber 22 of the container.

At this point the extraction tube 1 is positioned in a special housing 9 of an automatic machine for immunological tests and then the bottom cap 6 is removed so as to allow removal of the buffer solution 8 containing the faeces samples by a probe of the automatic machine.

It should be noted that the dome shape 41 of the upper part of the top cap 4 is designed to be substantially similar to the bottom of test tubes typically used in automatic analysers for clinical chemistry and immunological tests, to allow insertion of the extraction tube 1 inside the housing 9 of the automatic test machine.

Numerous changes and variations of detail within the reach of a person skilled in the art can be made to the present embodiment of the invention, without thereby departing from the scope of the invention as set forth in the appended claims.

## Claims

1. An extraction tube (1) for collection of faeces samples comprising:
- a container body (2), hollow on the inside, open at the top, and able to receive a buffer solution,
- a top cap (4) provided with a threaded small rod (49) for collection of faeces samples, said threaded small rod protruding axially inside the container body, when the top cap (4) is applied to the top end of the container body (2),
- a dividing partition (21) provided, in an intermediate position, inside said container body (2) so as to separate a top chamber (22) from a bottom chamber (23) inside said container body, said dividing partition (21) having an axial hole (26) suitable to allow the passage of said threaded small rod (49), so as to retain the excess faeces in said top chamber (22) and allow the passage of the threaded part of the small rod into said bottom chamber (23),
**characterised in that** said container body is open at the bottom and provided with a bottom cap (6) which can be applied movably to the bottom end of the container body, so that said extraction tube can be used directly as a primary sampling tube to be inserted in a sample-holder plate of automatic analysers, following removal of said bottom cap (6) and overturning of said container body (2) for positioning in a housing (9) of the automatic analyser, with the top cap (4) facing downward.

2. An extraction tube (1) according to claim 1, **characterised in that** said top cap (4) has an upper portion (41) destined to be disposed outside said container body (2), said upper portion (41) having a substantially domed shape similar to the bottom of a test tube normally used in the sample-holder plate of automatic analysers.

3. An extraction tube according to claim 1 or 2, **characterised in that** said top cap (4) comprises a stem (46) ending in an annular flange (47) beneath which is provided a cylindrical tang (48) surrounded by a seal (48') beneath which said threaded small rod (49) protrudes axially, and said dividing partition (21) comprising an annular flange (24) from which a cylindrical tang (25) protrudes axially upward so that when said top cap (4) is applied to the top end of the container body, the seal 48' of the cylindrical tang (48) of the stem (46) of the top cap is housed sealably inside a tapered part (26') of said hole (26) in the dividing partition.

4. An extraction tube (1) according to any one of the preceding claims, **characterised in that** said top cap (4) comprises a bottom portion (42) having an external thread (45) able to engage in an internal thread (27) provided in the top inner part of said container body (2).

5. An extraction tube (1) according to any one of the preceding claims, **characterised in that** said top cap (4) comprises a middle portion (40) having knurling (42) consisting of a plurality of longitudinal ribs and grooves.

6. An extraction tube (1) according to claims 4 and 5, **characterised in that** between said knurled part (44) of the top cap and said threaded part (45) of the top cap there is provided an annular seal (55) able to abut sealably on the top edge of said container body 2.

7. An extraction tube (1) according to any one of the preceding claims, **characterised in that** said top cap (4) has a blind hole (43) axially open at the top to the outside of the container body, and hexagonal in shape in cross section.

8. An extraction tube (1) according to any one of the preceding claims, **characterised in that** said bottom cap (6) has a top portion (61) with a plurality of annular collars (65) protruding radially outwards, to be able to be applied by pressure into the bottom part of said container body (2).

9. An extraction tube (1) according to any one of the preceding claims, **characterised in that** said bottom cap (6) comprises a middle portion (60) with knurling (64) consisting of a plurality of longitudinal ribs and grooves.

10. An extraction tube (1) according to any one of the preceding claims, **characterised in that** said bottom cap (6) has a blind hole (63) axially open at the top to the inside of the container body (2).

11. An extraction tube (1) according to any one of the preceding claims, **characterised in that** said threaded small rod (49) has in its bottom portion a thread consisting of a plurality of concentric collars (50) so as to define a plurality of annular spaces (51) therebetween, able to retain the sampled faeces.

## Patentansprüche

1. Auszugsröhre (1) zum Sammeln von Stuhlproben, welche umfasst:
- einen Behälterkörper (2), welcher im Innern hohl, an der Oberseite offen ist, und welcher in der Lage ist, eine Pufferlösung aufzunehmen,
- ein oberes Abschlussteil (4), welches einen mit Gewinde versehenen dünnen Stab (49) zum Sammeln der Stuhlproben aufweist, wobei der genannte, mit Gewinde versehene dünne Stab in axialer Richtung in das Innere des Behälterkörpers hineinragt, wenn das obere Abschlussteil (4) auf das obere Ende des Behälterkörpers (2) aufgesetzt wird,
- eine unterteilende Trennwand (21), welche in einer Zwischenstellung im Innern des genannten Behälterkörpers (2) derart angeordnet ist, dass sie im Innern des genannten Behälterkörpers eine obere Kammer (22) von einer unteren Kammer (23) trennt, wobei die genannte unterteilende Trennwand (21) eine in axialer Richtung verlaufende Öffnung (26) aufweist, welche so ausgelegt ist, dass sie das Hindurchführen des genannten, mit Gewinde versehenen dünnen Stabes (49) derart ermöglicht, dass das überschüssige Stuhlmaterial in der genannten oberen Kammer (22) zurückgehalten wird und das Einführen des mit Gewinde versehenen Teiles des dünnen Stabes in die genannte untere Kammer (23) möglich ist,
**dadurch gekennzeichnet, dass** der genannte Behälterkörper an der Unterseite offen ist und mit einem unteren Abschlussteil (6) versehen ist, welches lose auf das untere Ende des Behälterkörpers aufgesetzt werden kann, so dass die genannte Auszugsröhre direkt als primäres Probenahmerohr benutzt werden kann, welches - nach dem Entfernen des genannten unteren Abschlussteils (6) und dem Umkippen des genannten Behälterkörpers (2) zum Zweck seiner Unterbringung mit nach unten gerichtetem oberen Abschlussteil (4) in einem Gehäuse (9) eines automatischen Analysengerätes - in eine Probenhalterungsplatte eines automatischen Analysengerätes einzusetzen ist.

2. Auszugsröhre (1) gemäß Anspruch 1, **dadurch gekennzeichnet, dass** das genannte obere Abschlussteil (4) einen oberen Bereich (41) aufweist, welcher dafür vorgesehen ist, außerhalb des genannten Behälterkörpers (2) angeordnet zu werden, wobei der genannte obere Bereich (41) eine im Wesentlichen nach außen gewölbte Form ähnlich dem Bodens eines Reagenzglases aufweist, welches normalerweise in der Probenhalterungsplatte von automatischen Analysengeräten verwendet wird.

3. Auszugsröhre gemäß Anspruch 1 oder 2, **dadurch gekennzeichnet, dass** das genannte obere Abschlussteil (4) einen Schaft (46) umfasst, welcher in einem ringförmigen Flansch (47) endet, unterhalb welchem ein zylindrischer Mitnehmer (48) vorhanden ist, der von einer Dichtung (48') umgeben ist, unterhalb welcher der genannte, mit Gewinde versehene dünne Stab (49) in axialer Richtung herausragt, und dass die genannte unterteilende Trennwand (21) einen ringförmigen Flansch (24) umfasst, von welchem ein zylindrischer Mitnehmer (25) axial nach oben dergestalt absteht, dass, wenn das genannte obere Abschlussteil (4) auf das obere Ende des Behälterkörpers aufgesetzt wird, die Dichtung (48') des zylindrischen Mitnehmers (48) des Schaftes (46) des oberen Abschlussteiles abdichtend im Inneren eines konisch verlaufenden Teiles (26') der genannten Öffnung (26) in der unterteilenden Trennwand Sitz findet.

4. Auszugsröhre (1) gemäß einem der vorherigen Ansprüche, **dadurch gekennzeichnet, dass** das genannte obere Abschlussteil (4) einen unteren Bereich (42) umfasst, welcher ein Außengewinde (45) aufweist, welches in der Lage ist, in ein Innengewinde (27) eingeführt zu werden, welches sich am oberen inneren Teil des genannten Behälterkörpers (2) befindet.

5. Auszugsröhre (1) gemäß einem der vorherigen Ansprüche, **dadurch gekennzeichnet, dass** das genannte obere Abschlussteil (4) einen mittleren Bereich (40) umfasst, welcher eine Rändelung (42) aufweist, die aus einer Anzahl von in Längsrichtung verlaufenden Rippen und Riefen besteht.

6. Auszugsröhre (1) gemäß den Ansprüchen 4 und 5, **dadurch gekennzeichnet, dass** zwischen dem genannte gerändelten Teil (44) des oberen Abschlussteiles und dem genannten, mit Gewinde versehenen Teil (45) des oberen Abschlussteiles eine ringförmige Dichtung (55) angeordnet ist, welche abdichtend gegen den oberen Rand des genannten Behälterkörpers (2) stoßen kann.

7. Auszugsröhre (1) gemäß einem der vorherigen Ansprüche, **dadurch gekennzeichnet, dass** das genannte obere Abschlussteil (4) ein Blindloch (43) aufweist, welches in axialer Richtung am oberen Teil nach der Außenseite des Behälterkörpers hin offen ist und eine hexagonale Querschnittsform aufweist.

8. Auszugsröhre (1) gemäß einem der vorherigen Ansprüche, **dadurch gekennzeichnet, dass** das genannte untere Abschlussteil (6) einen oberen Bereich (61) mit einer Anzahl von ringförmigen Bunden (65) aufweist, welche in radialer Richtung nach außen gerichtet sind, damit dieses Abschlussteil unter Druckeinwirkung in den unteren Teil des genannten Behälterkörpers (2) eingesetzt werden kann.

9. Auszugsröhre (1) gemäß einem der vorherigen Ansprüche, **dadurch gekennzeichnet, dass** das genannte untere Abschlussteil (6) einen mittleren Bereich (60) mit einer Rändelung (64) umfasst, welche aus einer Anzahl von in Längsrichtung verlaufenden_Rippen und Riefen besteht.

10. Auszugsröhre (1) gemäß einem der vorherigen Ansprüche, **dadurch gekennzeichnet, dass** das genannte untere Abschlussteil (6) ein Blindloch aufweist, welches in axialer Richtung am oberen Teil nach der Innenseite des Behälterkörpers (2) hin offen ist.

11. Auszugsröhre (1) gemäß einem der vorherigen Ansprüche, **dadurch gekennzeichnet, dass** der mit Gewinde versehene dünne Stab (49) an seinem unteren Teil ein Gewinde aufweist, welches aus einer Anzahl von konzentrischen Bunden (50) dergestalt besteht, dass dazwischen zahlreiche ringförmige Räume (51) festgelegt werden, die ein Zurückbehalten des gesammelten Stuhlmaterials ermöglichen.

## Revendications

1. Un tube d'extraction (1) pour le recueil d'échantillons de fèces comprenant :
- un corps de récipient (2), creux à l'intérieur, ouvert à la partie supérieure, et susceptible de recevoir une solution tampon,
- un capuchon supérieur (4) présentant une petite tige filetée (49) pour le recueil d'échantillons de fèces, ladite petite tige filetée faisant saillie axialement à l'intérieur du corps de récipient, lorsque le capuchon supérieur (4) est appliqué sur l'extrémité supérieure du corps de récipient (2),
- une cloison diviseuse (21) prévue, dans une position intermédiaire, à l'intérieur dudit corps de récipient (2) de manière à séparer une chambre supérieure (22) d'une chambre inférieure (23) à l'intérieur dudit corps de récipient, ladite cloison diviseuse (21) présentant un trou axial (26) approprié pour permettre le passage de ladite petite tige filetée (49), de manière à retenir l'excès de fèces dans ladite chambre supérieure (22) et à permettre le passage de la partie filetée de la petite tige dans ladite chambre inférieure (23),
**caractérisé en ce que** ledit corps du récipient est ouvert à la partie inférieure et est muni d'un capuchon inférieur (6) qui peut être appliqué de manière mobile sur l'extrémité inférieure du corps de récipient, de sorte que ledit tube d'extraction peut être utilisé directement en tant que tube d'échantillonnage primaire devant être inséré dans une plaque porte-échantillon d'analyseurs automatiques, à la suite du retrait dudit capuchon inférieur (6) et du retournement dudit corps de récipient (2) pour être mis en place dans un boîtier (9) de l'analyseur automatique, avec le capuchon supérieur (4) dirigé vers le bas.

2. Un tube d'extraction (1) selon la revendication 1, **caractérisé en ce que** ledit capuchon supérieur (4) présente une partie supérieure (41) destinée à être disposée à l'extérieur dudit corps de récipient (2), ladite partie supérieure (41) présentant une forme sensiblement en dôme semblable à la partie inférieure d'un tube à essai normalement utilisé dans la plaque porte-échantillon d'analyseurs automatiques.

3. Un tube d'extraction selon la revendication 1 ou 2, **caractérisé en ce que** ledit capuchon supérieur (4) comprend une tige (46) se terminant en une bride annulaire (47) en dessous de laquelle est prévue une queue cylindrique (48) entourée par un joint (48') en dessous duquel ladite petite tige filetée (49) fait saillie axialement, et **en ce que** ladite cloison diviseuse (21) comprend une bride annulaire (24) à partir de laquelle une queue cylindrique (25) fait saillie axialement vers le haut de sorte que, lorsque ledit capuchon supérieur (4) est appliqué à l'extrémité supérieure du corps de récipient, le joint (48') de la queue cylindrique (48) de la tige (46) du capuchon supérieur est logé de manière étanche à l'intérieur d'une partie tronconique (26') dudit trou (26) formé dans la cloison diviseuse.

4. Un tube d'extraction (1) selon l'une quelconque des revendications précédentes, **caractérisé en ce que** ledit capuchon supérieur (4) comprend une partie inférieure (42) présentant un filetage externe (45) susceptible de s'engager dans un filetage interne (27) prévu dans la partie interne supérieure dudit corps de récipient (2).

5. Un tube d'extraction (1) selon l'une quelconque des revendications précédentes, **caractérisé en ce que** ledit capuchon supérieur (4) comprend une partie médiane (40) présentant un moletage (42) se composant d'une pluralité de nervures et de rainures longitudinales.

6. Un tube d'extraction (1) selon les revendications 4 et 5, **caractérisé en ce que**, entre ladite partie moletée (44) du capuchon supérieur et ladite partie filetée (45) du capuchon supérieur, il est prévu un joint annulaire (55) susceptible de venir en butée de manière étanche sur le bord supérieur dudit corps de récipient (2).

7. Un tube d'extraction (1) selon l'une quelconque des revendications précédentes, **caractérisé en ce que** ledit capuchon supérieur (4) présente un trou borgne (43) ouvert axialement à la partie supérieure vers l'extérieur du corps de récipient, et de forme hexagonale en section transversale.

8. Un tube d'extraction (1) selon l'une quelconque des revendications précédentes, **caractérisé en ce que** ledit capuchon inférieur (6) comprend une partie supérieure (61) présentant une pluralité de bagues annulaires (65) faisant saillie radialement vers l'extérieur, afin de pouvoir être appliquées par pression dans la partie inférieure dudit corps de récipient (2).

9. Un tube d'extraction (1) selon l'une quelconque des revendications précédentes, **caractérisé en ce que** ledit capuchon, inférieur (6) comprend une partie médiane (60) avec un moletage (64) se composant d'une pluralité de nervures et de rainures longitudinales.

10. Un tube d'extraction (1) selon l'une quelconque des revendications précédentes, **caractérisé en ce que** ledit capuchon inférieur (6) présente un trou borgne (63) ouvert axialement à la partie supérieure vers l'intérieur du corps de récipient (2).

11. Un tube d'extraction (1) selon l'une quelconques des revendications précédentes, **caractérisé en ce que** ladite petite tige filetée (49) présente, dans sa partie inférieure, un filetage se composant d'une pluralité de bagues concentriques (50) de manière à définir une pluralité d'espaces annulaires (51) entre eux, susceptibles de retenir les fèces échantillonnées.
